# EUROPÄISCHE PATENTSCHRIFT

(11) **EP 2 820 118 B1**
(45) Veröffentlichungstag und Bekanntmachung des Hinweises auf die Patenterteilung: **09.01.2019**
(21) Anmeldenummer: 13707818.4
(22) Anmeldetag: 01.03.2013
(51) Int. Cl.: C12M 1/12, C12M 1/26, C12M 1/34, C12Q 1/18

(54) **TEST-APPARATUR ZUR TESTUNG DER MIKROBIELLEN AKTIVITÄT AUF OBERFLÄCHEN**
TEST APPARATUS FOR TESTING THE MICROBIAL ACTIVITY ON SURFACES
APPAREIL DE TEST D'ACTIVITÉ MICROBIENNE SUR DES SURFACES

(30) Priorität: 02.03.2012 DE 102012004028
(43) Veröffentlichungstag der Anmeldung: 07.01.2015
(73) Patentinhaber: Universitätsklinikum Freiburg, 79104 Freiburg (DE)
(72) Erfinder: STEINBERG, Thorsten, 68259 Mannheim (DE); AL-AHMAD, Ali, 79117 Freiburg (DE); LIENKAMP, Karen, 79194 Freiburg (DE); NANKO, Norbert, 79108 Freiburg (DE)
(74) Vertreter: Dr. Langfinger & Partner
(86) Internationale Anmeldenummer: PCT/EP2013/000605
(87) Internationale Veröffentlichungsnummer: WO 2013/127536

(56) Entgegenhaltungen:
- CN-U- 202 011 880
- DE-A1- 10 328 556
- DE-A1- 19 758 598
- DE-A1-102005 038 737
- US-A- 4 146 433

## Beschreibung

Die vorliegende Erfindung betrifft eine Vorrichtung, umfassend eine Probenaufnahmeeinrichtung, eine Aufnahmeneinrichtung für Prüforganismen, vorzugsweise für eine Suspension umfassend Prüforganismen, insbesondere für eine Bakteriensuspension, und eine Strahlpumpeinrichtung, wobei die Strahlpumpeinrichtung in Wirkverbindung mit der Aufnahmeeinrichtung steht oder bringbar ist, und wobei die Strahlpumpeinrichtung ausgelegt und eingerichtet ist, um mittels eines Treibmedium mit einem höheren Druck als der Luftdruck am Aufstellort der Vorrichtung Prüforganismen in Form eines Aerosol in Richtung der Probeaufnahmeeinrichtung zu versprühen.

Biomaterialien werden in der Medizin für therapeutische oder diagnostische Zwecke eingesetzt. Diese können dabei in unmittelbaren Kontakt mit biologischem Gewebe des Körpers kommen und treten dabei in physikalische, chemische und/oder biologische Wechselwirkung mit den entsprechenden biologischen Systemen. Beispielsweise werden Biomaterialien im Bereich von Gewebezüchtungen (Tissue Engineering) sowie in verschiedenen Gebieten der medizinischen und zahnmedizinischen Implantologie eingesetzt.

Für den Einsatz von Biomaterialien müssen diese bestimmte Voraussetzungen erfüllen. Dazu zählen die Biokompatibilität und biologische Abbaubarkeit sowie die Begünstigung des Wachstums und der Differenzierung körpereigener Zellen. Demgegenüber stellt eine bakterielle Kontamination von Biomaterialen eine große postoperative Gefahr für Patienten dar. Eine solche bakterielle Kontamination gilt es daher nach Möglichkeit zu vermeiden.

Bei Biomaterialen und auch auf anderen Gebieten der Medizintechnik, zum Beispiel bei Oberflächenbeschichtungen medizinischer Geräte, sowie in weiteren Anwendungsgebieten, beispielsweise bei Wasseraufbereitungsanlagen, sind somit Materialien mit antiadhäsiven bzw. antimikrobiellen Oberflächen vorteilhaft.

Es ist im Stand der Technik bekannt, durch eine Modifikation von Materialoberflächen mit antimikrobiellen Komponenten, so genannten "synthetic mimics of antimicrobial peptides", antimikrobielle Biomaterialien zu erhalten.

Zur Überprüfung der Wirksamkeit einer solchen Behandlung von Materialoberflächen sind im Stand der Technik zwei unterschiedliche Testverfahren bekannt. Bei "Aqueous Antibacterial Assays" wird die zu überprüfende Oberfläche mit einer Suspension von Bakterien kontaktiert, bei "Airborne Antibacterial Assays" werden Bakterien-Aerosole in Kontakt mit der Oberfläche gebracht.

Bei "Aqueous Antibacterial Assays" wird eine Testoberfläche mit einer Prüforganismen umfassenden Suspension inokuliert und mit einem Deckglas bedeckt. Das Deckglas wird nach einer gewissen Kontaktzeit mit einem Puffer gespült und die resultierende Lösung wird inkubiert. Im Anschluss wird die Anzahl der gebildeten Bakterienkolonien bestimmt.

Eine Vorrichtung für ein standardisiertes Testverfahren für "Aqueous Antibacterial Assays" ist beispielsweise aus der DE 197 51 581 A1 bekannt. Dabei wird offenbart, dass eine Werkstoffprobe bereitgestellt werden soll, die mit einer Lösung, die den zu testenden Mikroorganismus enthält, inkubiert wird. Im Anschluss soll die Probe in ein für den entsprechenden Mikroorganismus geeignetes Minimalmedium überführt werden und danach in eine für den Mikroorganismus geeignete Nährlösung. Danach erfolgt die Entnahme der Probe und die messtechnische Erfassung der Nährlösung.

Auch die DE 197 58 598 A1 beschreibt ein Testverfahren für "Aqueous Antibacterial Assays", dass eine reproduzierbare Vermessung von Oberflächen ermöglichen soll.

Nachteilig an "Aqueous Antibacterial Assays" ist, dass beim Übergang der Bakterien vom Deckglas Fehler auftreten können, die das Messergebnis beeinflussen. Beispielsweise können Kontaminationen der Deckglasränder mit Bakteriensuspensionen auftreten, die nicht im direkten Kontakt mit der zu untersuchenden Oberfläche standen. Zudem kann eine unvollständige Ablösung von oberflächenadherierenden Zellen auftreten.

Im Unterschied dazu ist der "Airborne Antibacterial Assay" eine Simulation der Bakterienübertragung durch Tröpfcheninfektion. Dies stellt oftmals eine realitätsnähere Kontamination der zu untersuchenden Oberflächen dar. Bei dem "Airborne Antibacterial Assays" wird eine bakterielle Suspension auf die zu untersuchende Oberfläche mit einem Sprühgerät gesprüht.

Die DE 10 2005 038 737 A1 beschreibt eine gattungsgemäße Vorrichtung und ein Verfahren zum Auftragen von Prüforganismen auf eine Oberfläche. Dabei werden Prüforganismen mittels eines handelsüblichen Handsprühgeräts auf eine zu testende Oberfläche aufgetragen.

Nachteilig an der gattungsgemäßen Vorrichtung für "Airborne Antibacterial Assays" ist, dass kein reproduzierbares Vermessen von Oberflächen möglich ist. Durch den Einsatz von Handsprühgeräten variieren das Sprühvolumen der Bakteriensuspension, der Sprühabstand und der Sprühdruck.

DE10328556 offenbart eine Prüfkammer zur Untersuchung des mikrobiellen Wachstums auf oder in Probenkörpern. Die beschriebene Prüfkammer umfasst Probenaufnahmeeinrichtungen, eine Aufnahmeeinrichtung für eine Prüforganismen enthaltende Suspension und eine Beregnungseinrichtung, wobei die Beregnungseinrichtung in Wirkverbindung mit der Aufnahmeeinrichtung steht.

Des Weiteren ist es problematisch, dass humanpathogene Krankheitserreger mit der gattungsgemäßen Vorrichtung nicht getestet werden können. Durch den Einsatz von Handsprühgeräten besteht die Gefahr einer Kontamination des Laborbereichs mit den humanpathogenen Krankheitserregern, die vermieden werden muss.

Demgemäß wäre es wünschenswert, auf eine Vorrichtung zurückgreifen zu können, die ein standardisiertes Überprüfen von Materialeigenschaften mittels der "Airborne Antibacterial Assays" ermöglicht.

Demzufolge lag der vorliegenden Erfindung die Aufgabe zugrunde, die die Nachteile des Stands der Technik überwindet. Insbesondere soll eine Vorrichtung geliefert werden, die eine reproduzierbare Überprüfung der Eigenschaften von Materialoberflächen im Hinblick auf Prüforganismen ermöglicht. Des weiteren soll eine Vorrichtung geliefert werden, die eine Überprüfung der Materialeigenschaften von Oberflächen im Hinblick auf Bakteriensuspensionen, insbesondere humanpathogene Krankheitserreger, ermöglicht.

Die Aufgabe wird dadurch gelöst, dass die Vorrichtung eine Einrichtung zur Steuerung eines reproduzierbaren Drucks des Treibmediums während des Versprühens der Prüforganismen aufweist.

Durch einen reproduzierbaren Druck des Treibmediums kann sichergestellt werden, dass ein gleichmäßiges Versprühen der Prüforganismen auf die zu untersuchende Oberfläche einer Probe ermöglicht wird. Unter einem reproduzierbaren Druck soll unter anderem ein konstanter Druck, eine gesteuerte Änderung des Drucks, ein gewünschter Druckverlauf während eines Messvorgangs und/oder dergleichen verstanden werden.

Dies hat insbesondere den Vorteil, dass reproduzierbare Messungen der Wirkung der Prüforganismen auf die Proben möglich sind, so dass insbesondere mikrobiologische Untersuchungen von Oberflächen von unterschiedlichen zu untersuchenden Proben vergleichbar sind.

Es wird durch eine erfindungsgemäße Vorrichtung somit eine Standardisierung von Versuchsabläufen durch kontrollierte Versuchsbedingungen ermöglicht, die zudem eine schnellere und kostengünstigere Untersuchung der Proben ermöglicht.

Die Einrichtung zur Steuerung eines reproduzierbaren Drucks des Treibmediums umfasst ein Ventil zur Regulierung der Zuführung des Fluids, wobei vorzugsweise Druckluft durch einen Druckluftgeber (9) bereitgestellt wird.

Als Treibmedium hat sich die Verwendung eines gas- und/oder dampfförmigen Fluids als besonders vorteilhaft erwiesen. Die Verwendung eines flüssigen Treibmediums könnte gegebenenfalls zu einer zu starken Verdünnung der zu versprühenden Prüforganismen, insbesondere der Bakteriensuspension, führen.

Insbesondere die Verwendung von Druckluft und der Einsatz eines Druckluftgebers kann sich als vorteilhaft erweisen, um ein Treibmedium bereitzustellen. Druckluft und Druckluftgeber sind oftmals bereits vorhanden und können direkt verwendet werden.

Auch kann es vorteilhaft sein, dass eine Messeinrichtung zur Messung des zugeführten Volumens des Treibmediums umfasst ist, insbesondere einen Druckluftgeber umfassend eine Messeinrichtung zur Messung des zugeführten Luftvolumens.

Eine solche Messeinrichtung ermöglicht eine Regulierung des Treibmediums und eine Messung des zugeführten Volumens des Treibmediums, so dass eine hohe Reproduzierbarkeit des Versprühens der Prüforganismen ermöglicht wird.

Erfindungsgemäß kann es auch vorgesehen sein, dass ein Abstandeinstellmittel zum Einstellen eines reproduzierbaren Abstands der Probenaufnahmeeinrichtung von der Strahlpumpeinrichtung umfasst ist, vorzugsweise eine Schiene oder dergleichen, insbesondere umfassend eine Abstandsmesseinrichtung zum Messen des Abstands der Probenaufnahmeeinrichtung von der Strahlpumpeinrichtung.

Mittels eines Abstandeinstellmittels kann der Abstand von der Probenaufnahmeeinrichtung von der Strahlpumpeinrichtung reproduzierbar festgelegt werden. Die Anzahl der auf eine Probe versprühten Prüforganismen ist neben dem Druck des Treibmediums und anderen Faktoren auch vom Abstand der Strahlpumpeinrichtung von der Probenaufnahmeeinrichtung abhängig, so dass ein reproduzierbarer Abstand für reproduzierbare Messergebnisse vorteilhaft ist.

Dabei kann es auch vorgesehen sein, dass die Aufnahmeneinrichtung für Prüforganismen einen Erlenmeyerkolben umfasst und/oder ausbildet, vorzugsweise umfassend einen Einsatz für geringe Suspensionsvolumina, vorzugsweise einen Röhrcheneinsatz, insbesondere einen Reagenzglaseinsatz.

Der Einsatz von einem Erlenmeyerkolben als Aufnahmeneinrichtung für Prüforganismen ist vorteilhaft, da mit dieser durch den verjüngenden Hals eines Erlenmeyerkolbens ein unkontrolliertes Entweichen von Flüssigkeiten minimiert wird. Zudem kann dieser leicht dekontaminiert werden.

Auch kann vorgesehen sein, dass die Strahlpumpeinrichtung einen Chromatographie-Sprühaufsatz umfasst und/oder ausbildet.

Ein Chromatographie-Sprühaufsatz ermöglicht ein gerichtetes Versprühen der Prüforganismen und kann leicht dekontaminiert werden.

Dabei kann vorgesehen sein, dass die Probenaufnahmeeinrichtung eine Befestigungseinrichtung zum Befestigen eines zu untersuchenden Materials umfasst, insbesondere einen Substrathalter.

Eine solche Befestigungseinrichtung, insbesondere ein Substrathalter hat sich zum sicheren Befestigen der zu untersuchenden Proben als vorteilhaft erwiesen.

Auch kann vorgesehen sein, dass die Aufnahmeeinrichtung in Wirkverbindung mit einem Magnetrührer und/oder einem Heizrührer steht, insbesondere über einem Magnetrührer und/oder Heizrührer angeordnet ist, wobei vorzugsweise ein Magnet, insbesondere ein stabförmiger Magnet, der in Wirkverbindung mit dem Magnetrührer und/oder dem Heizrührer steht, in der Aufnahmeeinrichtung angeordnet ist, wobei das Gehäuse insbesondere hermetisch verschlossen ist.

Mittels einem Magnetrührer und/oder einem Heizrührer kann eine gute Durchmischung der Prüforganismen insbesondere in einer Suspension sichergestellt werden, so dass vorteilhafterweise eine homogene Verteilung der Prüforganismen in der Suspension erreicht wird.

Auch kann vorgesehen sein, dass die Vorrichtung ein Gehäuse umfasst, insbesondere eine Kammer, vorzugsweise eine Inokulationskammer, wobei zumindest die Probenaufnahmeeinrichtung, die Aufnahmeneinrichtung für Prüforganismen, die Strahlpumpeinrichtung, der Magnetrührer und/oder der Heizrührer und/oder die Mittel zum Einstellen eines reproduzierbaren Abstands von Probenaufnahmeeinrichtung und Strahlpumpeinrichtung innerhalb des Gehäuses angeordnet ist bzw. sind.

Ein solches Gehäuse ermöglicht einem Benutzer den guten Regeln der Laborarbeiten zu entsprechen, in dem die Ausbreitung von Aerosolen verhindert wird. Vorteilhafterweise kann durch die Verwendung eines solchen Gehäuses der Benutzer vor den verwendeten Prüforganismen geschützt werden. Mittels eines Einsatzes eines solchen Gehäuses können zudem auch Humanpathogene als Prüforganismen eingesetzt werden.

Dabei kann vorgesehen sein, dass das Gehäuse einen zumindest teilweise transparenten Bereich ausbildet, wobei insbesondere der Bereich teilweise aus Glas und/oder Plexiglas besteht.

Ein solcher transparenter Bereich ermöglicht die Überwachung des Versprühens der Prüforganismen auf die zu untersuchende Oberfläche der Probe.

Auch kann es vorteilhaft sein, dass das Gehäuse zumindest teilweise, insbesondere vollständig, aus einem temperaturbeständigen Kunststoff, insbesondere Polyetherketon (PEEK), und/oder Aluminium, insbesondere eloxiertem Aluminium, besteht.

Ein Gehäuse, dass zumindest teilweise aus einem temperaturbeständigen Kunststoff, insbesondere Polyetherketon (PEEK), und/oder Aluminium besteht, kann einfach dekontaminiert werden. Beispielsweise ist eine Dekontamination eines solchen Gehäuses in einem Autoklaven möglich. Dies reduziert den Zeitaufwand und die Kosten einer Dekontamination signifikant.

Des weiteren kann es vorteilhaft sein, dass das Gehäuse zumindest ein Druckausgleichmittel für einen, insbesondere kontaminationsfreien, Druckausgleich zwischen dem Druck in dem Gehäuse und dem Luftdruck am Aufstellort umfasst, insbesondere einen Filter zum Filtern der Abluft, vorzugsweise einen, insbesondere autoklavierbaren, Teflonfilter.

Ein solches Druckausgleichmittel ist vorteilhaft, um zu verhindern, dass ein Überdruck innerhalb des Gehäuses entsteht. Durch die Strahlpumpe erhöht sich das Fluidvolumen innerhalb des Gehäuses, das zur Vermeidung einer Kontamination des Benutzers vorteilhafterweise insbesondere hermetisch verschlossen ist. Durch einen Einsatz eines Filters kann ein kontaminationsfreier Druckausgleich vom Inneren des Gehäuses mit dem Umgebungsdruck durch das Druckausgleichmittel sichergestellt werden.

Auch kann vorgesehen sein, dass das Gehäuse eine Einrichtung zum Öffnen und Schließen des Gehäuses umfasst, vorzugsweise eine Klappe, Tür und/oder dergleichen, wobei die Einrichtung zumindest teilweise aus einem transparenten Material besteht.

Eine solche Einrichtung ermöglicht ein einfaches Einbringen und Entnehmen der Proben, der Prüforganismen, der Einstellung des Abstands von Probeaufnahmeeinrichtung und Aufnahmeeinrichtung für Prüforganismen und/oder dergleichen.

Schließlich kann auch vorgesehen sein, dass das Gehäuse einen Anschluss für einen Druckluftgeber umfasst.

Ein solcher Anschluss ist vorteilhaft, da Druckluft oftmals zentral bereitgestellt wird und somit einfach nutzbar ist.

Auch liefert die Erfindung einer Verwendung einer erfindungsgemäßen Vorrichtung zur Simulation einer Übertragung von Prüforganismen durch Tröpfcheninfektion auf eine Oberfläche, insbesondere einer Bakterienübertragung, vorzugsweise von humanpathogenen Keimen, vorzugsweise zum Testen einer antimikrobiellen Aktivität einer Oberfläche.

Der vorliegenden Erfindung lag die überraschende Erkenntnis zugrunde, dass mittels einer erfindungsgemäßen Vorrichtung ein standardisiertes Überprüfen von Oberflächeneigenschaften im Hinblick auf Prüforganismen bereitgestellt werden kann. Dies kann erfindungsgemäß dadurch erreicht werden, dass eine Strahlpumpe zum Versprühen von Prüforganismen verwendet wird, die insbesondere in Form einer Suspension vorliegen. Für reproduzierbare Messergebnisse wird dabei das Treibmittel der Strahlpumpe mit nahezu konstantem Druck zugeführt und es kann ein definierter Abstand zwischen der Strahlpumpe und einer Probenaufnahmeeinrichtung eingestellt und vorzugsweise erfasst werden. Dabei können insbesondere auch Humanpathogene getestet werden.

Weitere Merkmale und Vorteile der Erfindung ergeben sich aus der nachfolgenden Beschreibung, in der Ausführungsbeispiele der Erfindung anhand von schematischen Zeichnungen beispielhaft erläutert werden, ohne dadurch die Erfindung zu beschränken. Dabei zeigt:

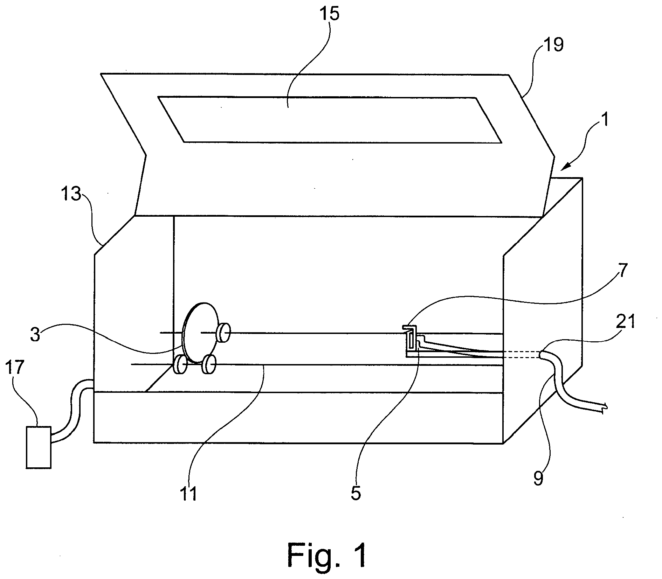

Figur 1: eine schematische Perspektivansicht einer erfindungsgemäßen Vorrichtung.

In Figur 1 ist eine schematische Perspektivansicht einer erfindungsgemäßen Vorrichtung 1 gezeigt. Die Vorrichtung 1 umfasst dabei eine Probenaufnahmeneinrichtung 3, eine Aufnahmeeinrichtung 5 für Prüforganismen und eine Strahlpumpeinrichtung 7. Die Aufnahmeeinrichtung 5 ist dabei vorzugsweise für die Aufnahme von Suspensionen umfassend der Prüforganismen geeignet. Die Strahlpumpeinrichtung 7 ist dabei derart angeordnet, dass sie der Probenaufnahmeneinrichtung 3 gegenüber liegt. Ein Druckluftgeber 9 stellt dabei ein Treibmedium für die Strahlpumpeinrichtung 7 bereit.

Der Abstand zwischen der Strahlpumpeinrichtung 7 und der Probenaufnahmeeinrichtung 3 kann dabei mittels eines Abstandeinstellmittel 11 eingestellt werden. Dabei ist insbesondere vorgesehen, dass das Abstandeinstellmittel 11 eine Messeinrichtung umfasst (nicht gezeigt), um den besagten Abstand zum messen und/oder anzuzeigen.

Des weiteren umfasst die Vorrichtung 1 ein Gehäuse 13 mit einem transparenten Bereich 15. In dem Gehäuse 13 sind dabei insbesondere die Probenaufnahmeneinrichtung 3, die Aufnahmeeinrichtung 5 für Prüforganismen und die Strahlpumpeinrichtung 7 angeordnet. Dabei kann es selbstverständlich vorgesehen sein, dass weitere Einrichtungen der Vorrichtung 1 (nicht gezeigt) innerhalb des Gehäuses 1 angeordnet sind.

Das Gehäuse 13 ist dabei vorzugsweise hermetisch verschließbar, so dass keine Prüforganismen beim Versprühen eben dieser mittels der Strahlpumpeinrichtung 7 in die Umgebung des Gehäuses 13 gelangen können.

Um einen Überdruck innerhalb des Gehäuses 13 zu verhindern ist ein Druckausgleichmittel 17, insbesondere ein Filter oder dergleichen, an dem Gehäuse 13 angeordnet. Mittels des Druckausgleichsmittels kann somit ein Druckausgleich erfolgen und gleichzeitig können die in der Umgebung unerwünschten Prüforganismen herausgefiltert werden.

Mittels einer Klappe 19 wird ein einfacher Zugang zum Inneren des Gehäuses 13 bereitgestellt. Für eine Zuführung des Treibmittels ist zudem ein Anschluss 21 vorgesehen, insbesondere ein Anschluss 21 für einen Druckluftschlauch.

Für ein standardisiertes Überprüfen von Oberflächeneigenschaften im Hinblick auf Prüforganismen können diese zunächst an der Probenaufnahmeneinrichtung 3 angeordnet werden. Die insbesondere in einer Suspension vorliegenden Prüforganismen werden in die Aufnahmeeinrichtung 5 eingebracht. Die Strahlpumpe 7 wird anschließend in Wirkverbindung mit der mit der Suspension befüllten Aufnahmeeinrichtung 5 gebracht. Im Anschluss wird mittels des Abstandeinstellmittels 11 der gewünschte Abstand zwischen der Aufnahmeeinrichtung 5 und der Strahlpumpe 7 sowie der Probenaufnahmeneinrichtung 3 eingestellt. Um eine unerwünschte Verteilung der während des Versprühens der Suspension entstehenden Aerosole zu vermeiden, wird die Klappe 19 geschlossen. Eine weitere Beobachtung der Abläufe ist durch den transparenten Bereich 15 möglich.

Durch eine Einstellung des Drucks und des Zuführvolumens des Treibmittels mittels des Druckgebers 9 kann nunmehr eine kontrollierte und reproduzierte Beaufschlagung der Probe in der Probenaufnahmeneinrichtung 3 erfolgen. Ein entstehender Überdruck innerhalb der Kammer 13 wird durch das Druckausgleichsmittel 17 kompensiert.

Die in der vorstehenden Beschreibung sowie in den Ansprüchen offenbarten Merkmale der Erfindung können sowohl einzeln als auch in jeder beliebigen Kombination für die Verwirklichung der Erfindung in ihren verschiedenen Ausführungsformen wesentlich sein.

### Bezugszeichenliste

- 1: Vorrichtung
- 3: Probenaufnahmeeinrichtung
- 5: Aufnahmeneinrichtung
- 7: Strahlpumpeinrichtung
- 9: Druckluftgeber
- 11: Abstandeinstellmittel
- 13: Gehäuse
- 15: Bereich
- 17: Druckausgleichmittel
- 19: Klappe
- 21: Anschluss

## Patentansprüche

1. Vorrichtung (1), umfassend eine Probenaufnahmeeinrichtung (3), eine Aufnahmeeinrichtung (5) für Prüforganismen, vorzugsweise für eine Suspension umfassend Prüforganismen, insbesondere für eine Bakteriensuspension, und eine Strahlpumpeinrichtung (7), wobei die Strahlpumpeinrichtung (7) in Wirkverbindung mit der Aufnahmeeinrichtung steht oder bringbar ist, und wobei die Strahlpumpeinrichtung (7) ausgelegt und eingerichtet ist, um mittels eines Treibmedium mit einem höheren Druck als der Luftdruck am Aufstellort der Vorrichtung (1) Prüforganismen in Form eines Aerosol in Richtung der Probeaufnahmeeinrichtung zu versprühen, **dadurch gekennzeichnet, dass**
die Vorrichtung (1) eine Einrichtung zur Steuerung eines reproduzierbaren Drucks des Treibmediums während des Versprühens der Prüforganismen aufweist, und wobei die Einrichtung zur Steuerung eines reproduzierbaren Drucks des Treibmediums ein Ventil zur Regulierung der Zuführung des Fluids umfasst, und wobei vorzugsweise Druckluft durch einen Druckluftgeber (9) bereitgestellt wird.

2. Vorrichtung (1) nach Anspruch 1, **dadurch gekennzeichnet, dass**
eine Messeinrichtung zur Messung des zugeführten Volumens des Treibmediums umfasst ist, insbesondere einen Druckluftgeber (9) umfassend eine Messeinrichtung zur Messung des zugeführten Luftvolumens.

3. Vorrichtung (1) nach einem der Ansprüche 1 oder 2, **dadurch gekennzeichnet, dass**
ein Abstandeinstellmittel (11) zum Einstellen eines reproduzierbaren Abstands der Probenaufnahmeeinrichtung (3) von der Strahlpumpeinrichtung (7) umfasst ist, vorzugsweise eine Schiene oder dergleichen, insbesondere umfassend eine Abstandsmesseinrichtung zum Messen des Abstands der Probenaufnahmeeinrichtung (3) von der Strahlpumpeinrichtung (7).

4. Vorrichtung (1) nach einem der vorangehenden Ansprüche, **dadurch gekennzeichnet, dass**
die Probenaufnahmeeinrichtung (5) für Prüforganismen einen Erlenmeyerkolben umfasst und/ oder ausbildet, vorzugsweise umfassend einen Einsatz für geringe Suspensionsvolumina, vorzugsweise einen Röhrcheneinsatz, insbesondere ein Reagenzglaseinsatz.

5. Vorrichtung (1) nach einem der vorangehenden Ansprüche, **dadurch gekennzeichnet, dass**
die Strahlpumpeinrichtung (7) einen Chromatographie-Sprühaufsatz umfasst und/oder ausbildet.

6. Vorrichtung (1) nach einem der vorangehenden Ansprüche, **dadurch gekennzeichnet, dass**
die Probenaufnahmeeinrichtung (3) einen Befestigungseinrichtung zum Befestigen eines zu untersuchenden Materials umfasst, insbesondere einen Substrathalter.

7. Vorrichtung (1) nach einem der vorangehenden Ansprüche, **dadurch gekennzeichnet, dass**
die Aufnahmeeinrichtung in Wirkverbindung mit einem Magnetrührer und/oder einem Heizrührer steht, insbesondere über einem Magnetrührer und/oder Heizrührer angeordnet ist, wobei vorzugsweise ein Magnet, insbesondere ein stabförmiger Magnet, der in Wirkverbindung mit dem Magnetrührer und/oder dem Heizrührer steht, in der Aufnahmeeinrichtung angeordnet ist.

8. Vorrichtung (1) nach einem der vorangehenden Ansprüche, **dadurch gekennzeichnet, dass** die Vorrichtung (1) ein Gehäuse (13) umfasst, insbesondere eine Kammer, vorzugsweise eine Inokulationskammer, wobei zumindest die Probenaufnahmeeinrichtung (3), die Probenaufnahmeeinrichtung (5) für Prüforganismen, die Strahlpumpeinrichtung (7), der Magnetrührer und/oder der Heizrührer und/oder die Mittel zum Einstellen eines reproduzierbaren Abstands von Probenaufnahmeeinrichtung (3) und Strahlpumpeinrichtung (7) innerhalb der Gehäuse (13)s angeordnet ist bzw. sind, wobei das Gehäuse (13) insbesondere hermetisch verschlossen ist.

9. Vorrichtung (1) nach Anspruch 8, **dadurch gekennzeichnet, dass** das Gehäuse (13) einen zumindest teilweise transparenten Bereich (15) ausbildet, wobei insbesondere der Bereich (15) teilweise aus Glas und/oder Plexiglas besteht.

10. Vorrichtung (1) nach einem der Ansprüche 8 oder 9, **dadurch gekennzeichnet, dass** das Gehäuse (13) zumindest teilweise, insbesondere vollständig, aus einem temperaturbeständigen Kunststoff, insbesondere Polyetherketon (PEEK), und/oder Aluminium, insbesondere eloxiertem Aluminium, besteht.

11. Vorrichtung (1) nach einem der Ansprüche 8 bis 10, **dadurch gekennzeichnet, dass** das Gehäuse (13) zumindest ein Druckausgleichmittel (17) für einen, insbesondere kontaminationsfreien, Druckausgleich zwischen dem Druck in dem Gehäuse (13) und dem Luftdruck am Aufstellort umfasst, insbesondere einen Filter zum Filtern der Abluft, vorzugsweise einen, insbesondere autoklavierbaren, Teflonfilter.

12. Vorrichtung (1) nach einem der Ansprüche 8 bis 11, **dadurch gekennzeichnet, dass** das Gehäuse (13) einen Einrichtung (19) zum Öffnen und Schließen des Gehäuse (13)s umfasst, vorzugsweise eine Klappe, Tür und/oder dergleichen, wobei die Einrichtung zumindest teilweise aus einem transparenten Material besteht.

13. Vorrichtung (1) nach einem der Ansprüche 8 bis 12, **dadurch gekennzeichnet, dass** das Gehäuse (13) einen Anschluss für einen Druckluftgeber (9) umfasst.

14. Verwendung einer Vorrichtung (1) nach einem der Ansprüche 1 bis 13 zur Simulation einer Übertragung von Prüforganismen durch Tröpfcheninfektion auf eine Oberfläche, insbesondere einer Bakterienübertragung, vorzugsweise von humanpathogenen Keimen, vorzugsweise zum Testen einer antimikrobiellen Aktivität einer Oberfläche.

## Claims

1. Device (1) comprising a sample accommodation mechanism (3), an accommodation mechanism (5) for test organisms, preferably for a suspension comprising test organisms, more particularly for a bacterial suspension, and a jet pump mechanism (7), wherein the jet pump mechanism (7) is or is bringable in active connection with the accommodation mechanism, and wherein the jet pump mechanism (7) is designed and set up to spray test organisms in the form of an aerosol in the direction of the sample accommodation mechanism by means of a propellant having a higher pressure than the air pressure at the installation site of the device (1), **characterized in that**
the device (1) has a mechanism for the controlling of a reproducible pressure of the propellant during the spraying of the test organisms, and wherein the mechanism for the controlling of a reproducible pressure of the propellant comprises a valve for the regulation of the supply of the fluid, and wherein compressed air is preferably provided by means of a compressed air provider (9).

2. Device (1) according to Claim 1, **characterized in that** a measurement mechanism for the measurement of the supplied volume of the propellant is comprised, more particularly a compressed air provider (9) comprising a measurement mechanism for the measurement of the supplied air volume.

3. Device (1) according to either of Claims 1 and 2, **characterized in that** a distance setting mechanism (11) for the setting of a reproducible distance of the sample accommodation mechanism (3) from the jet pump mechanism (7) is comprised, preferably a track or the like, more particularly comprising a distance measurement mechanism for the measurement of the distance of the sample accommodation mechanism (3) from the jet pump mechanism (7).

4. Device (1) according to any of the preceding claims, **characterized in that** the sample accommodation mechanism (5) for test organisms comprises and/or forms an Erlenmeyer flask, preferably comprising an insert for low suspension volumes, preferably a tube insert, more particularly a test tube insert.

5. Device (1) according to any of the preceding claims, **characterized in that** the jet pump mechanism (7) comprises and/or forms a chromatography spray attachment.

6. Device (1) according to any of the preceding claims, **characterized in that** the sample accommodation mechanism (3) comprises an attachment mechanism for the attachment of a material to be investigated, more particularly a substrate holder.

7. Device (1) according to any of the preceding claims, **characterized in that** the accommodation mechanism is in active connection with a magnetic stirrer and/or a heating stirrer, more particularly is arranged above a magnetic stirrer and/or heating stirrer, wherein a magnet, more particularly a bar-shaped magnet, which is in active connection with the magnetic stirrer and/or the heating stirrer is preferably arranged in the accommodation mechanism.

8. Device (1) according to any of the preceding claims, **characterized in that** the device (1) comprises a housing (13), more particularly a chamber, preferably an inoculation chamber, wherein at least the sample accommodation mechanism (3), the sample accommodation mechanism (5) for test organisms, the jet pump mechanism (7), the magnetic stirrer and/or the heating stirrer and/or the means for the setting of a reproducible distance between sample accommodation mechanism (3) and jet pump mechanism (7) is/are arranged within the housing (13), wherein the housing (13) is more particularly hermetically sealed.

9. Device (1) according to Claim 8, **characterized in that** the housing (13) forms an at least partly transparent region (15), wherein more particularly the region (15) consists partly of glass and/or plexiglass.

10. Device (1) according to either of Claims 8 and 9, **characterized in that** the housing (13) consists at least partly, more particularly completely, of a temperature-resistant plastic, more particularly polyether ketone (PEEK), and/or aluminium, more particularly anodized aluminium.

11. Device (1) according to any of Claims 8 to 10, **characterized in that** the housing (13) comprises at least one pressure equalizer (17) for a pressure equalization, more particularly contamination-free pressure equalization, between the pressure in the housing (13) and the air pressure at the installation site, more particularly a filter for the filtering of the waste air, preferably a Teflon filter, more particularly autoclavable Teflon filter.

12. Device (1) according to any of Claims 8 to 11, **characterized in that** the housing (13) comprises a mechanism (19) for the opening and closing of the housing (13), preferably a flap, door and/or the like, wherein the mechanism consists at least partly of a transparent material.

13. Device (1) according to any of Claims 8 to 12, **characterized in that** the housing (13) comprises a port for a compressed air provider (9).

14. Use of a device (1) according to any of Claims 1 to 13 for the simulation of a transmission of test organisms by droplet infection onto a surface, more particularly a bacterial transmission, preferably of human pathogens, preferably for the testing of an antimicrobial activity of a surface.

## Revendications

1. Dispositif (1), comprenant un équipement de logement de sonde (3), un équipement de logement (5) pour des organismes de test, de préférence pour une suspension comprenant des organismes de test, en particulier pour une suspension de bactéries, et un équipement de pompe à jet (7), l'équipement de pompe à jet (7) se trouvant ou pouvant être mis en liaison fonctionnelle avec l'équipement de logement, et l'équipement de pompe à jet (7) étant étudié et conçu pour pulvériser au moyen d'un agent propulseur à une pression supérieure à la pression de l'air dans le lieu d'installation du dispositif (1) des organismes de test sous forme d'un aérosol en direction de l'équipement de logement de sonde, **caractérisé en ce que** le dispositif (1) comporte un équipement destiné à la commande d'une pression reproductible de l'agent propulseur pendant la pulvérisation des organismes de test, et l'équipement destiné à la commande d'une pression reproductible de l'agent propulseur comprenant une soupape destinée à la régulation de l'introduction du fluide, et l'air comprimé étant fourni de préférence par un injecteur d'air comprimé (9).

2. Dispositif (1) selon la revendication 1, **caractérisé en ce qu'**il comprend un équipement de mesure destiné à la mesure du volume d'agent propulseur introduit, en particulier un injecteur d'air comprimé (9) comprenant un équipement de mesure destiné à la mesure du volume d'air introduit.

3. Dispositif (1) selon l'une quelconque des revendications 1 et 2, **caractérisé en ce qu'**il comprend un moyen de réglage de distance (11) destiné à régler une distance reproductible entre l'équipement de logement de sonde (3) et l'équipement de pompe à jet (7), de préférence un rail ou similaire, comprenant en particulier un équipement de mesure de distance destiné à mesurer la distance entre l'équipement de logement de sonde (3) et l'équipement de pompe à jet (7).

4. Dispositif (1) selon l'une quelconque des revendications précédentes, **caractérisé en ce que** l'équipement de logement de sonde (5) pour des organismes de test comprend et/ou constitue un erlenmeyer, comprenant de préférence un insert pour de petits volumes de suspension, de préférence un insert de petits tubes, en particulier un insert d'éprouvette.

5. Dispositif (1) selon l'une quelconque des revendications précédentes, **caractérisé en ce que** l'équipement de pompe à jet (7) comprend et/ou constitue un embout de pulvérisation de chromatographie.

6. Dispositif (1) selon l'une quelconque des revendications précédentes, **caractérisé en ce que** l'équipement de logement de pompe (3) comprend un équipement de fixation destiné à fixer un matériau à étudier, en particulier un porte-substrat.

7. Dispositif (1) selon l'une quelconque des revendications précédentes, **caractérisé en ce que** l'équipement de logement se trouve en liaison fonctionnelle avec un agitateur magnétique et/ou un agitateur thermique, en particulier est agencé au-dessus d'un agitateur magnétique et/ou d'un agitateur thermique, un aimant, en particulier un aimant en forme de barre, qui se trouve en liaison fonctionnelle avec l'agitateur magnétique et/ou avec l'agitateur thermique étant de préférence agencé dans l'équipement de logement.

8. Dispositif (1) selon l'une quelconque des revendications précédentes, **caractérisé en ce que** le dispositif (1) comprend un boîtier (13), en particulier une chambre, de préférence une chambre d'inoculation, au moins l'équipement de logement de sonde (3), l'équipement de logement de sonde (5) pour organismes de test, l'équipement de pompe à jet (7), l'agitateur magnétique et/ou l'agitateur thermique et/ou les moyens destinés à régler une distance reproductible entre l'équipement de logement de sonde (3) et l'équipement de pompe à jet (7), étant agencé (s) à l'intérieur du boîtier (13), le boîtier (13) étant en particulier fermé hermétiquement.

9. Dispositif selon la revendication 8, **caractérisé en ce que** le boîtier (13) constitue une zone (15) au moins partiellement transparente, la zone (15) étant en particulier constituée au moins partiellement de verre et/ou de plexiglas.

10. Dispositif (1) selon l'une quelconque des revendications 8 ou 9, **caractérisé en ce que** le boîtier (13) est constitué au moins partiellement, en particulier totalement, d'un plastique résistant à la chaleur, en particulier de polyéthercétone (PEEK), et/ou d'aluminium, en particulier d'aluminium anodisé.

11. Dispositif (1) selon l'une quelconque des revendications 8 à 10, **caractérisé en ce que** le boîtier (13) comprend au moins un moyen de compensation de pression (17) pour une compensation de pression, en particulier sans contamination, entre la pression du boîtier (13) et la pression de l'air dans le lieu d'installation, en particulier un filtre destiné à filtrer l'air sortant, de préférence un filtre en téflon, pouvant en particulier être traité à l'autoclave.

12. Dispositif (1) selon l'une quelconque des revendications 8 à 11, **caractérisé en ce que** le boîtier (13) comprend un équipement (19) destiné à ouvrir et fermer le boîtier (13), de préférence un volet, une porte et/ou similaire, l'équipement étant constitué au moins partiellement d'un matériau transparent.

13. Dispositif (1) selon l'une quelconque des revendications 8 à 12, **caractérisé en ce que** le boîtier (13) comprend un raccordement pour un injecteur d'air comprimé (9).

14. Utilisation d'un dispositif (1) selon l'une quelconque des revendications 1 à 13 destiné à la simulation d'une transmission d'organismes de test par infection par gouttelettes sur une surface, en particulier d'une transmission de bactéries, de préférence de germes de pathogènes humains, de préférence pour tester une activité antimicrobienne d'une surface.
